# EUROPEAN PATENT APPLICATION

(11) **EP 2 433 554 A1**
(43) Date of publication of application: **28.03.2012**
(21) Application number: 10777776.5
(22) Date of filing: 19.05.2010
(51) Int. Cl.: A61B 5/00

(54) **BIOLOGICAL INFORMATION MANAGEMENT DEVICE, HEALTH MANAGEMENT SYSTEM USING A BIOLOGICAL INFORMATION MANAGEMENT DEVICE, METHOD FOR BROWSING HEALTH MANAGEMENT INFORMATION IN SAID SYSTEM, AND BIOLOGICAL INFORMATION MANAGEMENT PROGRAM**

(30) Priority: 21.05.2009 JP 2009123498
(71) Applicant: NEC Corporation, Tokyo 108-8001 (JP)
(72) Inventor: ARIMITSU, Yoko, Tokyo 108-8001 (JP)
(74) Representative: Stork Bamberger
(86) International application number: PCT/JP2010/058441
(87) International publication number: WO 2010/134543

(57) **Abstract**

Provided is a system which enables more satisfactory health care closely tied with user's daily life and enables a user to execute health care without noticing and whose facility in use is improved. A health care system 1 is set up by biological information obtaining devices 10A and 10B, a biological information management device 20 which obtains user authentication by short-distance radio communication from the biological information obtaining device and receives biological information of a measured user, and a health care information providing device 30 connected to the biological information management device via a communication network 50 to receive biological information transmitted from the biological information management device, generate health care information by processing and transmit the generated information to the biological information management device as a requesting source. To the communication network, a health care information intermediating device 40 may be further connected.

## Description

### TECHNICAL FIELD

The present invention relates to a biological information management device which executes health care of a user by using a portable communication terminal, a health care system using the biological information management device, a method of reading health care information in the system, and a biological information management program.

### BACKGROUND ART

It has been common practice to obtain biological information such as body temperature, pulses, weight and body fat by measuring them by a user by means of a measuring apparatus for health care available at stores and execute health care by the user himself/herself based on the obtained biological information. Health care by such a method, however, largely depends on a user's intention, so that it is not always sufficient as a means for constantly monitoring the condition of one's health.

Under these circumstances, Patent Literature 1, for example, proposes the health care supporting system in which a user sends biological information or health information measured by a biological information measuring device incorporated into a sanitary cleaning device to a medical facility or the like and the medical facility which receives the information generates health care advice information based on the collected information and transmits the same to the user.

Patent Literature 1: Japanese Patent Laid-Open No. 2004-13508.

According to the technique disclosed in the above-described Patent Literature 1, a cellular phone intermediates between a sensor incorporated into a sanitary cleaning device and a server disposed in a medical facility and a user manually inputs sensor measurement information to send the same to the server of the medical facility, thereby obtaining advice information. While Patent Literature 1 recites automating manual input of measurement information by a radio communication unit using infrared rays or the like, it fails to recite a procedure of communication with the medical facility including security in such a case.

The technique disclosed in Patent Literature 1 fails to cover management of biological information when a user is away from home.

Even in facilities such as a fitness club, a restaurant, a supermarket, a convenience store and a hospital, if a user is allowed to receive the above-described service, more satisfactory health care closely tied with daily life can be realized and a user's facility in use will be improved.

### (OBJECT OF THE INVENTION)

An object of the present invention is to provide a biological information management device which enables more satisfactory health care closely tied with a user's daily life and enables a user to execute health care without noticing and whose facility in use is improved, a health care system using the biological information management device, a method of reading health care information in the system, and a biological information management program.

### SUMMARY

According to a first exemplary aspect of the invention, a biological information management device includes a control unit which is connected to a biological information obtaining device via short-distance radio communication and connected to a health care information providing device via a communication network;
wherein the control unit receives biological information of a user measured after obtaining user authentication by the short-distance radio communication from the biological information obtaining device, and transmits the biological information received to the health care information providing device via the communication network, receives health care information which is processed by an analysis based on the biological information and transmitted from the health care information providing device and outputs the received information.

According to a second exemplary aspect of the invention, a health care system using a biological information management device, includes
a biological information obtaining device,
a biological information management device which receives biological information of a user measured after obtaining user authentication by short-distance radio communication from the biological information obtaining device, and
a health care information providing device connected to the biological information management device via a communication network to receive the biological information transmitted from the biological information management device, generate health care information by processing and transmit the generated information to the biological information management device as a requesting source.

According to a third exemplary aspect of the invention, a method of reading health care information in a health care system including a biological information obtaining device, a health care information providing device and a biological information management device which is connected to the biological information obtaining device via short-distance radio communication and connected to the health care information providing device via a communication network, wherein
the biological information management device includes
a first step of receiving biological information of a user measured after obtaining user authentication by the short-distance radio communication from the biological information obtaining device, and
a second step of transmitting the biological information received to the health care information providing device via the communication network,
the health care information providing device includes
a third step of processing biological information received from the biological information management device by an analysis and transmitting the generated health care information to the biological information management device or the health care information intermediating device, and
the biological information management device includes
a fourth step of receiving health care information transmitted from the health care information providing device to cause a user to read the information, or obtaining user authentication by short-distance radio communication from the health care information intermediating device and receiving health care information transmitted from the health care information intermediating device to cause the user to read the information.

According to a fourth exemplary aspect of the invention, a biological information management program executed on a computer which realizes a biological information management device connected to a biological information obtaining device via short-distance radio communication and connected to a health care information providing device via a communication network, which causes the computer to execute
a biological information reception processing of receiving biological information of a user measured after obtaining user authentication by the short-distance radio communication from the biological information obtaining device, and
a control processing of transmitting the biological information received to the health care information providing device via the communication network, receiving health care information which is processed by analyzing the biological information and transmitted from the health care information providing device and outputting the received information.

The present invention enables provision of a biological information management device which enables more satisfactory health care closely tied with a user's daily life and enables a user to execute health care without noticing and whose facility in use is improved, a health care system using the biological information management device, a method of reading health care information in the system, and a biological information management program.

The reason is that from a biological information obtaining device, a control unit receives a user's biological information measured upon obtaining user authentication by short-distance radio communication, transmits the same to a health care information providing device via a communication network, and receives and outputs health care information processed by analyses based on the biological information and transmitted from the health care information providing device.

### BRIEF DESCRIPTION OF THE DRAWINGS

Fig. 1 is a diagram showing a structure of a health care system and a data flow of an exemplary embodiment of the present invention;
Fig. 2 is a diagram showing an operation sequence of the health care system according to the present exemplary embodiment of the present invention;
Fig. 3 is a block diagram showing an internal structure of a biological information obtaining device of the health care system according to the present exemplary embodiment of the present invention;
Fig. 4 is a block diagram showing an internal structure of a biological information management device according to the exemplary embodiment of the present invention;
Fig. 5 is a block diagram showing a structure of a health care information providing device of the health care system according to the exemplary embodiment of the present invention;
Fig. 6 is a block diagram showing an internal structure of a health care information intermediating device of the health care system according to the exemplary embodiment of the present invention;
Fig. 7 is a flow chart showing operation of the biological information management device according to the exemplary embodiment of the present invention; and
Fig. 8 is a flow chart showing operation of the health care information providing device of the health care system according to the exemplary embodiment of the present invention.

### EXEMPLARY EMBODIMENT

Next, an exemplary embodiment of the present invention will be described in detail with reference to the drawings.

### (STRUCTURE OF FIRST EXEMPLARY EMBODIMENT)

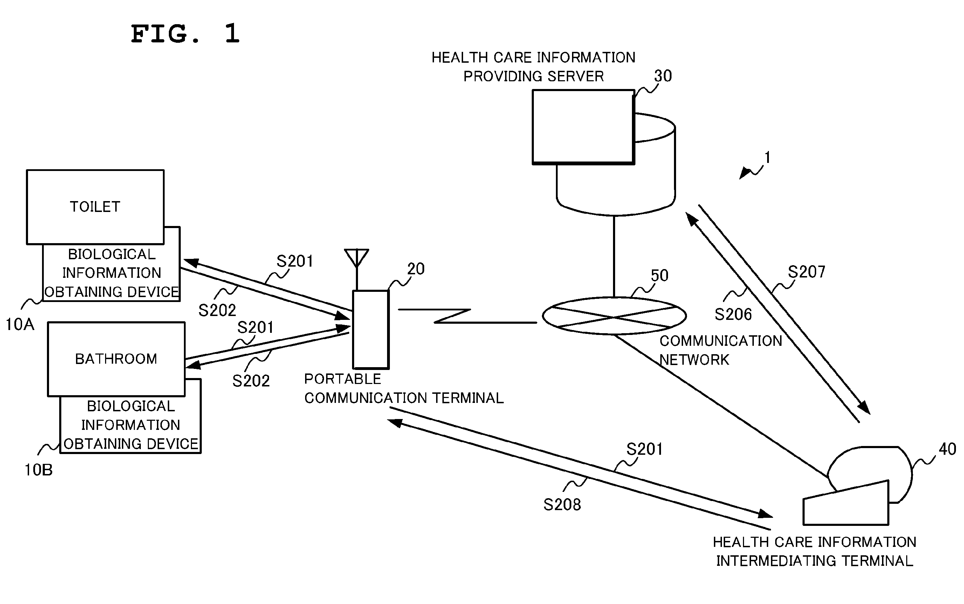

Fig. 1 is a block diagram showing a structure of a health care system 1 according to a first exemplary embodiment of the present invention.

With reference to Fig. 1, a health care system 1 according to the first exemplary embodiment of the present invention includes biological information obtaining devices 10A and 10B, a portable communication terminal 20 for use as a biological information management device of the present invention, and a health care information providing server 30 for use as a health care information providing device.

The above-described portable communication terminal 20 is connected to the health care information providing server 30 via a communication network 50 such as an IP (Internet Protocol) network. Connected to the communication network 30 may be a health care information intermediating terminal 40 equivalent to a PC (Personal Computer) for use as a health care information intermediating device.

The biological information obtaining devices 10A and 10B have a function of obtaining a user's biological information such as body temperature, pulses, weight, body fat and a urinary component measured by various kinds of sensors embedded in a toilet, a bathroom (bath tub) and the like which are disposed in a domestic life space and a function of transmitting the measured biological information to the portable communication terminal 20 through short-distance radio communication when the user holds up the portable communication terminal 20 to have user authentication established.

At the time of obtaining biological information, the portable communication terminal 20 carried by a user has a function of obtaining user authentication through short-distance radio communication from the biological information obtaining devices 10A and 10B, receiving biological information of the user to be measured and transmitting the information to the health care information providing server 30 via the communication network 50 and a function of obtaining health care information analyzed, processed and generated based on the biological information from the health care information providing server or the health care information intermediating terminal 40.

The health care information providing server 30 is managed and operated, for example, by a service provider including a medical facility. The health care information providing server 30 has a function of receiving a contracted user's biological information transmitted from the portable communication terminal 20 carried by the user, generating health care information (e.g. life improvement program information (information about recommendation for diet, exercise, etc.) according to biological information) by analysis and processing, and transmitting the generated information to the portable communication terminal 20 or the health care information intermediating terminal 40 as a requesting source.

The health care information intermediating terminal 40 is disposed at, for example, a fitness club, a restaurant, a supermarket, a convenience store, a hospital or the like. The health care information intermediating terminal 40 has a function of intermediating health care information based on biological information transmitted from the health care information providing server 30 via the communication network 50 after user authentication from the portable communication terminal 20 is established by short-distance radio communication and transmitting the information to the portable communication terminal 20 as a requesting source.

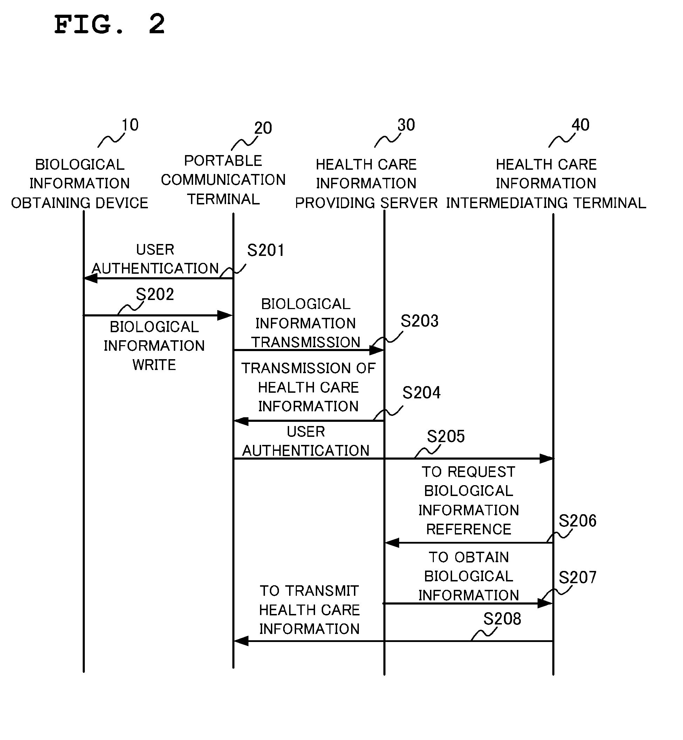

In the following, description will be made of a data flow of the health care system 1 shown in Fig. 1 with reference to the operation sequence diagram shown in Fig. 2.

With reference to Fig. 2, a user executes short-distance radio communication by holding up the portable communication terminal 20 over the biological information obtaining devices 10A and 10B when using a bath tub or a toilet at home. As a result, the portable communication terminal 20 obtains its own biological information by obtaining user authentication from the biological information obtaining device 10 and writes the same to a built-in memory (Steps S201 and S202). At this time, the portable communication terminal 20 accesses the health care information providing server 30 via the communication network 50 to transmit the formerly obtained biological information of its own (Step S203). It is at this time possible to transmit the measured biological information so as to be correlated with a user's position information.

Upon accumulation of a predetermined amount of a user's biological information or in response to a request from the user, the health care information providing server 30 responsively analyzes and processes the accumulated user's biological information to generate health care information such as the above-described life improvement program and transmit the same to the user's portable communication terminal 20 (Step S204). This enables the user to read these pieces of information displayed on a screen of the portable communication terminal 20 that he/she carries.

The user is allowed to receive the same service also when going out, for example at a fitness club. In this case, it is necessary to execute short-distance radio communication by holding up the portable communication terminal 20 over the health care information intermediating terminal 40 disposed at the fitness club to obtain user authentication (Step S205). When the user authentication is established, the health care information intermediating terminal 40 accesses the health care information providing server 30 (Step S206) to obtain the already accumulated user's biological information which is provided from the health care information providing server 30 or health care information such as a generated life improvement program (Step S207). Then, the health care information intermediating terminal 40 transmits the obtained health care information to the portable communication terminal 20 as a requesting source or transmits a plan of health improvement through fitness or the like which is uniquely analyzed and generated by the fitness club based on the obtained biological information or the like (Step S208).

Since when the health care information intermediating terminal 40 has a copy of a user's biological information of a fixed time period, the health care information intermediating terminal 40 need not obtain biological information through communication with the health care information providing server 30 every time when necessary, and it can quickly provide health care information readable by the user. On this occasion, an agreement between the fitness club and a provider which supplies health care information is required for making health care information open under a user's approval. Also by giving permission for reading to a trainer of the fitness club by a user's approval, an advice for improving health can be obtained efficiently in terms of fitness.

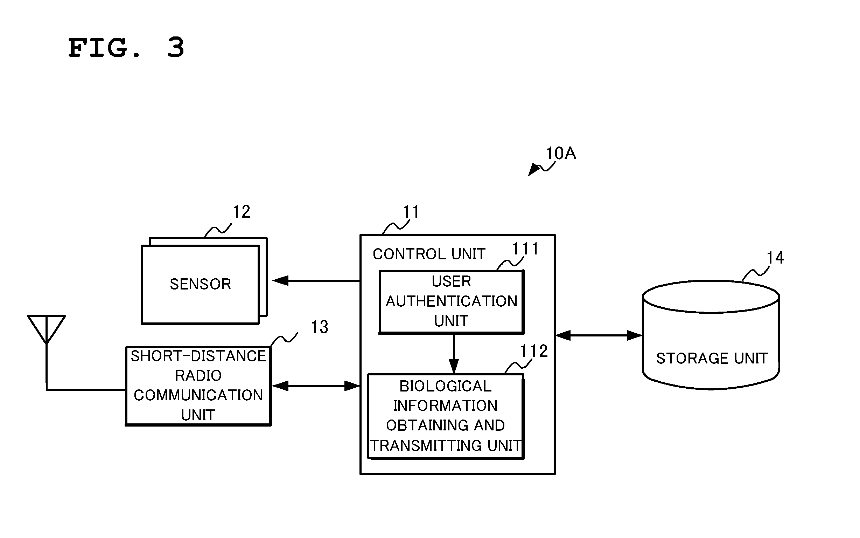

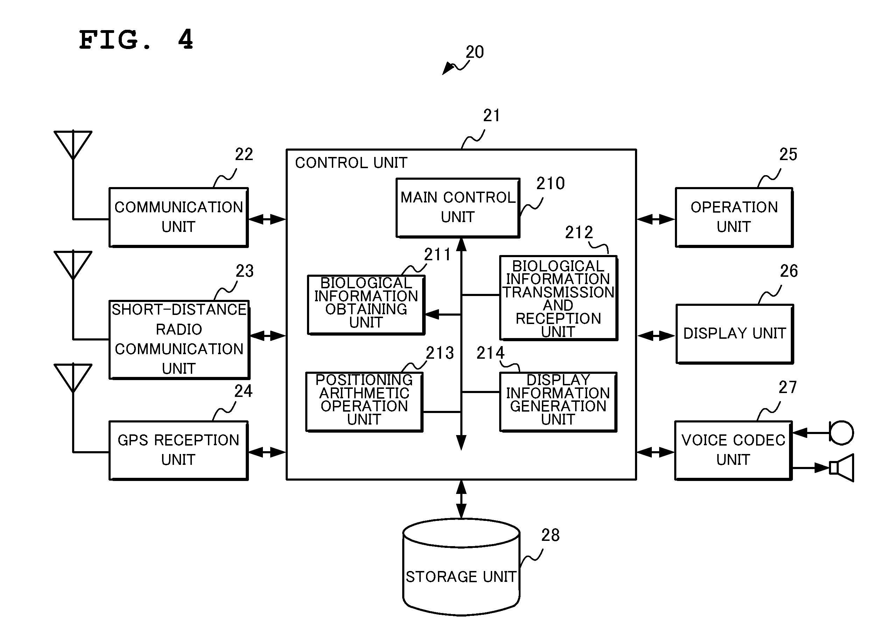

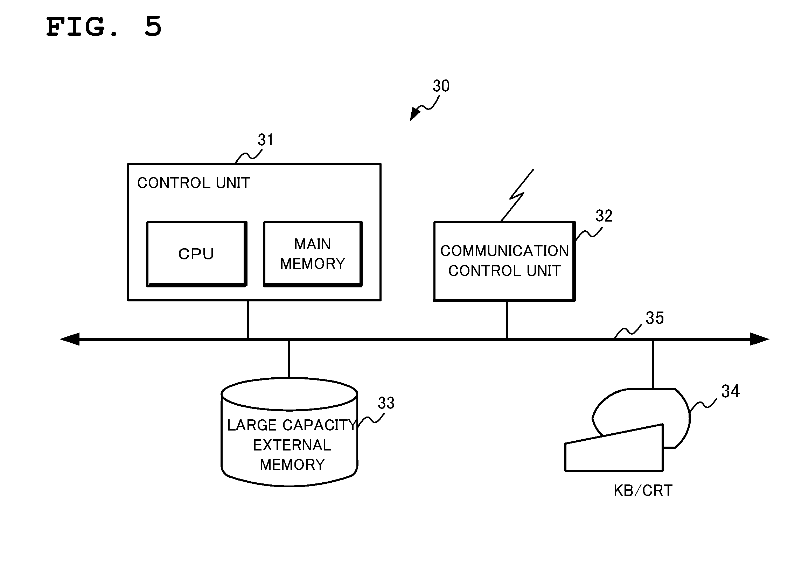

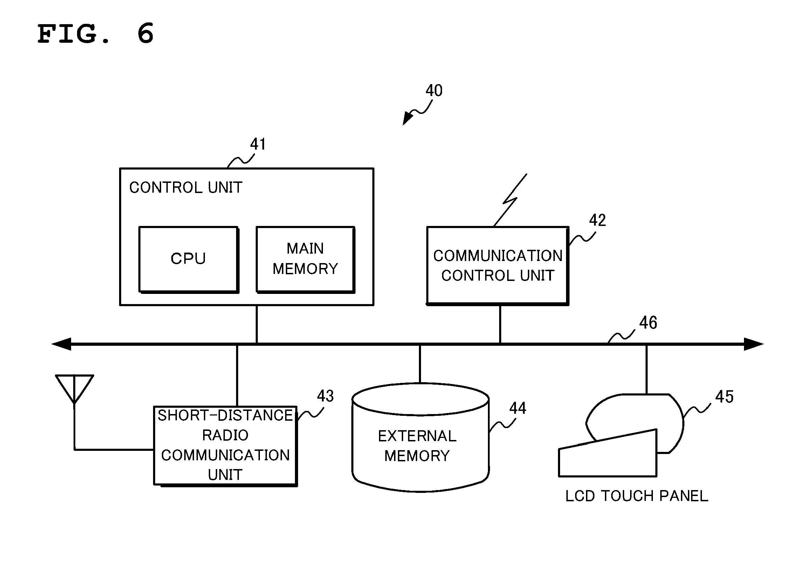

Next, detailed description will be made of each device forming the health care system 1 according to the present exemplary embodiment with reference to Fig. 3 through Fig. 6. Fig. 3 shows the structure of the biological information obtaining device 10A, Fig. 4 shows that of the portable communication terminal 20, Fig. 5 shows that of the health care information providing server 30 and Fig. 6 shows that of the health care information intermediating terminal 40.

With reference to Fig. 3, the biological information obtaining device 10A includes a control unit 11, a sensor 12, a short-distance radio communication unit 13 and a storage unit 14. The biological information obtaining device 10B also has the same structure.

The sensor 12 senses a user's biological information such as body temperature, pulses, weight, body fat and a urinary component and outputs the same to the control unit 11.

The short-distance radio communication unit 13 generates electromagnetic waves to execute short-distance radio communication with a short-distance radio communication unit 23 of the portable communication terminal 20 which will be described later. The short-distance radio communication unit 13, which internally includes a modulation/demodulation unit and a resonance circuit unit formed of a coil and a condenser, modulates and demodulates electromagnetic waves generated by a resonance frequency to execute non-contact communication with the portable communication terminal 20. The short-distance radio communication unit 13 only needs to be able to execute non-contact communication such as radio communication by a radio communication technique known as the Bluetooth (Registered Trademark), radio LAN (Local Area Network) or RFIC (Radio Frequency IC).

It is assumed here to use short-distance radio communication by RFIC built in the portable communication terminal 20 available at stores and it is accordingly assumed that the short-distance radio communication unit 13 of the biological information obtaining device 10A has a reader/writer built therein.

The control unit 11 is structured by a microprocessor and Fig. 3 shows a structure of a program executed by the microprocessor with its function expanded. As shown in the diagram, the control unit 11 includes a user authentication unit 111 and a biological information obtaining and transmitting unit 112.

The user authentication unit 111 is for recognizing identification information (information inherent to a user) recorded in the RFIC built in the portable communication terminal 20 by executing short-distance radio communication with the portable communication terminal 20 through the short-distance radio communication unit 13. Here, as long as the read identification information is identification information already registered at the storage unit 14, the biological information obtaining and transmitting unit 112 is activated, so that the biological information obtaining and transmitting unit 112 causes the sensor 12 to start measurement of biological information, and the measured biological information is transmitted to the portable communication terminal 20 through the short-distance radio communication unit 13 (reader writer) and written to the same. The measured biological information is recorded also in the storage unit 14.

With reference to Fig. 4, the portable communication terminal 20 includes a control unit 21, a communication unit 22, the short-distance radio communication unit 23, a GPS (Global Positioning System) reception unit 24, an operation unit 25, a display unit 26 and a voice CODEC (Coder Decoder) unit 27.

The communication unit 22 grasps the radio communication system and executes radio communication with a base station (not shown) connected to the communication network 50 to transmit or receive various kinds of data. Various kinds of data here represents voice data mainly at the time of voice communication, mail data at the time of mail transmission/reception, page data at the time of web browsing, etc. which includes a user's biological information.

Since the short-distance radio communication unit 23 is the same as the short-distance radio communication unit 13 of the biological information obtaining devices 10A and 1013, no description will be made of the same to avoid repetition of the description. The GPS reception unit 24 outputs current position information of the portable communication terminal 1 related to latitude, longitude and time which is received from a GPS satellite to the control unit 21. The current position information is used as information for positioning a current position for the purpose of a user's behavior tracing which is executed by the control unit 21.

The operation unit 25 has keys to which various kinds of functions are assigned such as a power key, a communication key, a number key, a character key, a direction key, an enter key, a transmission key and a function key and when such a key is operated by a user, generates a signal corresponding to contents of the operation and outputs the same to the control unit 21 as a user instruction.

The display unit 26 is structured using, for example, LCD (Liquid Crystal Display Device) or an organic EL (Electro-Luminescence) formed of numerous pixels (a combination of light emitting devices of a plurality of colors) arranged crosswise. The display unit 26 displays an image according to display target data such as a document generated by the control unit 21 and written in a predetermined region (VRAM region) of a storage unit 28.

The voice CODEC unit 27 executes input/output processing of a voice signal output from a speaker or a voice signal input through a microphone. More specifically, the voice CODEC unit 27 amplifies voice input through the microphone, executes analog-digital conversion of the same, further subjects the same to signal processing such as coding, converts the obtained signal into digital voice data and outputs the obtained data to the control unit 21. The voice CODEC unit 27 executes signal processing such as decoding of voice data supplied from the control unit 21, digital-analog conversion, amplification, etc., converts the obtained signal into an analog voice signal and outputs the obtained signal to the speaker.

The storage unit 28 stores various kinds of data for use in various kinds of processing of the portable communication terminal 20. Stored, for example, are a computer program executed by the control unit 21, various kinds of setting data and temporary data for use in the course of program processing. Further stored in the storage unit 28 is a user's biological information transmitted from the biological information obtaining devices 10A and 10B. The storage unit 28 is structured, for example, by a non-volatile storage device (a non-volatile semiconductor memory, a hard disk device, an optical disk device, etc.) or a random-access storage device (e.g. SRAM, or DRAM).

The control unit 21 executes general control of the entire operation of the portable communication terminal 20. More specifically, the unit controls operation of each of the above-described control blocks (signal transmission and reception at the communication unit 22, information transfer to/from the short-distance radio communication unit 23, acquisition of current position information from the GPS reception unit 24, take-in of operation input from the operation unit 25, display of an image at the display unit 26, voice input/output at the voice CODEC unit 27, etc.) such that various kinds of processing of the portable communication terminal 20 (voice communication executed through a circuit switching network, creation and transmission/reception of electronic mail, browsing of a Web (World Wide Web) site on the Internet) are executed by an appropriate procedure according to the operation of the operation unit 25.

The control unit 21, which comprises a computer (microprocessor) that executes processing based on a program (an operating system, an application program, etc.) stored in the storage unit 26, executes the above-described processing according to the procedure instructed by these programs. More specifically, the control unit 21 sequentially reads instruction codes from a program such as an operating system or an application program stored in the storage unit 28 and executes the same.

The control unit 21 also functions as a control unit which receives and stores a user's biological information measured after obtaining user authentication by short-distance radio communication from the biological information obtaining devices 10A and 10B, as well as transmitting the received biological information to the health care information providing server 30 via the communication network 50, receiving health care information processed and generated by an analysis based on biological information and transmitted from the health care information providing server 30, and outputting the received information.

For realizing the function as the above-described control unit, the control unit 21 includes a main control unit 210, a biological information obtaining unit 211, a biological information transmission and reception unit 212, a positioning arithmetical operation unit 213 and a display information generation unit 214 as shown in Fig. 4 which illustrates the structure of the program executed by the control unit 21 with its function expanded.

The biological information obtaining unit 211 has a function of obtaining biological information received through the short-distance radio communication unit 23 and writing the same to the storage unit 28. The biological information transmission and reception unit 212 has a function of transmitting the obtained biological information to the health care information providing server 30 via the main control unit 210, the communication unit 22 and the communication network 50 and a function of receiving health care information processed by an analysis based on the biological information and transmitted from the health care information providing server 30 to start display information generation processing by the display information generation unit 214. The biological information transmission and reception unit 212 also has a function of obtaining a user's authentication by short-distance radio communication from the health care information intermediating terminal 40 further connected through the communication network 50 and receiving health care information transmitted from the health care information providing server 30 via the health care information intermediating terminal 40 and outputting the same.

The positioning arithmetic operation unit 213 has a function of executing positioning arithmetic operation based on a user's current position information received at the GPS reception unit 24 and transferring the result to the main control unit 210. The main control unit 210 responsively correlates the result of the positioning arithmetic operation with the previously obtained biological information and transmits the correlation result to the health care information providing server 30 via the communication network 50. The display information generation unit 214 has a function of generating display information for displaying health care information transmitted from the health care information providing server 30 or the health care information intermediating terminal 40 on at least a part of a screen of the display unit 26 and of outputting the information.

Since the control unit 21 functions as a control unit which receives a user's biological information measured after obtaining user authentication by short-distance radio communication from the biological information obtaining devices 10A and 10B, as well as transmitting the received biological information to the health care information providing server 30 via the communication network 50, receiving health care information processed by an analysis based on the biological information and transmitted from the health care information providing server 30, and outputting the received information, the main control unit 210 executes sequence control of the above-described biological information obtaining unit 211, the biological information transmission and reception unit 212, the positioning arithmetic operation unit 213 and the display information generation unit 214.

With reference to Fig. 5, the health care information providing server 30 is formed of a control unit 31 including a CPU (Central Processing Unit) and a main memory, a communication control unit 32, a large capacity external memory 33 and a display input device (KB/CRT) 34. These are all connected in common via a system bus 35 formed of a plurality of lines for address, data and control.

The control unit 31 obtains, via the communication control unit 32, a user's biological information transmitted from the portable communication terminal 20 via the communication network 50 and accumulates the information at the large capacity external memory 33. The control unit 31, sequentially or when a fixed amount of biological information is accumulated in the large capacity external memory 33 or based on a request from the portable communication terminal 20, analyzes and processes the biological information to generate health care information such as a life improvement program and transmits the same to the portable communication terminal 20 via the communication control unit 32 and the communication network 50. The control unit 31 transmits a user's biological information accumulated in the large capacity external memory 33 or health care information such as a life improvement program to the health care information intermediating terminal 40 via the system bus 35, a communication control unit 42 and the communication network 50 based on a biological information reference request transmitted under user authentication from the health care information intermediating terminal 40.

The communication control unit 32 transmits and receives health care information including biological information by communication with the portable communication terminal 20 or the health care information intermediating terminal 40 according to, for example, a protocol such as TCP/IP (Transmission Control/Intemet Protocol). The large capacity external memory 33 is formed of a large capacity storage element such as an HDD or an optical disk which accumulates a user's biological information and the display input device (KB/CRT 34) is used at the time of editing when health care information such as a life improvement program is created.

With reference to Fig. 6, the health care information intermediating terminal 40 is formed of a control unit 41 including a CPU and a main memory, the communication control unit 42, a short-distance radio communication unit 43, an external memory 44 and an LCD touch panel 45. These are all connected in common via a system bus 46 formed of a plurality of lines for address, data and control.

When user authentication from the portable communication terminal 20 is established through short-distance radio communication using the short-distance radio communication unit 43, the control unit 41 receives health care information based on a user's biological information transmitted from the health care information providing server 30 via the communication network 50 and the communication control unit 42 and transmits the information to the portable communication terminal 20 as a requesting source through the system bus 46 and the short-distance radio communication unit 43.

The communication control unit 42 transmits and receives health care information including biological information through communication with the health care information providing server 30 based on TCP/IP, for example. The external memory 44 is formed of a storage element such as HDD which accumulates a user's biological information or health care information received from the health care information providing server 30 and the LCD touch panel 45 is used at the time of editing when health care information is generated for providing unique service.

### (OPERATION OF THE FIRST EXEMPLARY EMBODIMENT)

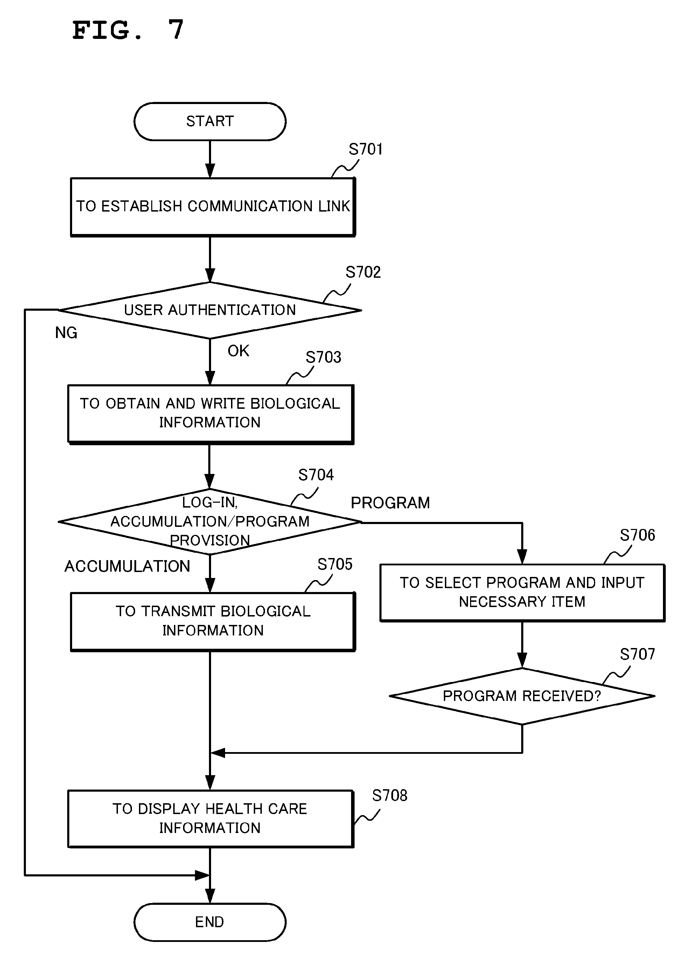

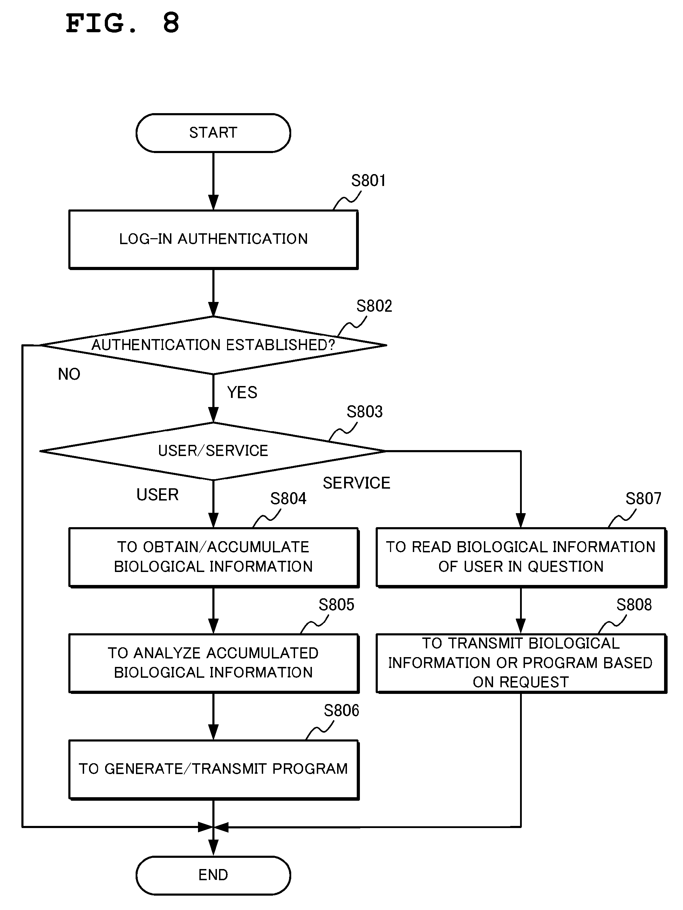

In the following, detailed description will be made of operation of the portable communication terminal 20 and the health care information providing server 30 according to the first exemplary embodiment with reference to the flow charts of Fig. 7 and Fig. 8.

Fig. 7 is a flow chart showing a flow of processing of the portable communication terminal 20. With reference to Fig. 7, when a user holds up the portable communication terminal 20 over the biological information obtaining device 10 when entering a toilet or a bathroom, a communication link is established between the biological information obtaining device 10 and the portable communication terminal 20 (Step S701). Then, execute user authentication by short-distance radio communication using RFIC (Step S702). More specifically, the user identification information recorded in the RFIC (short-distance radio communication unit 23) built in the portable communication terminal 20 is read by the reader/writer (short-distance radio communication unit 13) of the biological information obtaining device 10 and collated with user identification information stored in the storage unit 14 of the biological information obtaining device 10.

As a result of the above-described collation, when the identification information coincides with each other to establish user authentication by the user authentication unit 111 ("YES" at Step S702), a user's biological information (excretions, body temperature, weight, skin color, the state of swelling, etc.) is automatically sensed by the sensor 12 while the user is in the toilet or the bathroom. The biological information is written into the storage unit 28 of the portable communication terminal 20 under the control of the control unit 21 by the short-distance radio communication executed between the short-distance radio communication unit 13 of the biological information obtaining device 10 and the short-distance radio communication unit 23 of the portable communication terminal 20 (Step S703).

The control unit 21 of the portable communication terminal 20 logs in the health care information providing server 30 and when simply desiring only the accumulation of biological information ("accumulation" at Step 5704), transmits the user's biological information previously obtained by the biological information transmission and reception unit 212 to the health care information providing server 30 (Step S705). On the other hand, when desiring transmission of an analysis result ("program" at Step S704), the user operates the operation unit 25 to select a program and inputs a necessary item (Step S706) to wait for transmission of an analysis result including the program from the health care information providing server 30 (Step S707).

When health care information such as a life improvement program including the analysis result is transmitted from the health care information providing server 30 ("YES" at Step S707), at the portable communication terminal 20, the biological information transmission and reception unit 212 of the control unit 21 obtains the information via the communication network 50 and the communication unit 22, and the display information generation unit 214 generates display information based on health care information indicated by the obtained analysis result to display the same on the display unit 26 (Step S708). This enables the user to read the analysis result of the health condition based on the measured biological information. While the description has been made here of a case where, when a user desires transmission of an analysis result, the health care information providing server 30 analyzes a fixed amount of a user's accumulated biological information to transmit health care information, it is possible to analyze contents of biological information every time it is received and transmit an analysis result such as pulses, abnormal blood pressure or the like.

Fig. 8 is a flow chart showing a flow of processing of the health care information providing server 30. With reference to Fig. 8, when the portable communication terminal 20 or the health care information intermediating device 40 logs in (Step S801), the health care information providing server 30 executes user authentication. When the user authentication is established here ("YES" at Step S802), in a case of log-in by a user ("user" at Step S803), the control unit 31 accumulates biological information transmitted in the large capacity external memory 33 (Step S804). Then, the control unit 31 analyzes a current health condition sequentially or upon accumulation of a predetermined amount or based on a user's request (Step S805) to generate health care information such as an optimum life program, a life improvement program or the like by collating the current state with an ideal health condition and transmits the information to the portable communication terminal 20 as a requesting source (Step S806).

On the other hand, in a case of log-in from the health care information intermediating terminal 40 ("service" at Step S803), that is, when a user holds the portable communication terminal 20 over the health care information intermediating terminal 40 to execute user authentication and the authentication is established, the control unit 31 of the health care information providing server 30 reads biological information of the user in question from the large capacity external memory 33 (Step S807). Then, the control unit 31 transmits a user's biological information or a program already processed and generated by an analysis based on a request (Step S808).

The health care information intermediating terminal 40 having received the biological information or the health care information such as a life improvement program generated as a result of an analysis transmits the information to the portable communication terminal 20 of the user via the short-distance radio communication unit 43.

The portable communication terminal 20 having received the information displays the same at the display unit 26 or outputs the same as voice through a speaker by means of the voice CODEC unit 27.

It is also possible for the health care information intermediating terminal 40 to uniquely analyze a user's biological information received from the health care information providing server 30 to determine whether the health condition is good or bad, as well as, when improvement is necessary, extracting an item of behavior optimum for the user in diet, exercise, etc. to generate a unique fitness program and transmit the same to the portable communication terminal 20 by short-distance radio communication.

While in the above-described first exemplary embodiment, described is only a case where the health care information intermediating terminal 40 is disposed in a fitness club, it may be disposed in a restaurant, a supermarket, a convenience store, a hospital or the like other than a fitness club. In this case, unique service can be provided for each field in which the biological information is used. Biological information is obtained not exclusively from a toilet or a bathroom at home but in cooperation with a sensor embedded in a toilet outside the house, a bath tub or the like.

### (EFFECTS OF THE FIRST EXEMPLARY EMBODIMENT)

In the biological information management device (portable communication terminal 20) according to the present exemplary embodiment, the control unit 21 receives a user's biological information measured after obtaining user authentication from the biological information obtaining device 10A (10B) by short-distance radio communication, as well as transmitting the received biological information to the health care information providing server 30 via the communication network 50 and receiving health care information to be processed by an analysis based on the biological information and transmitted from the health care information providing server 30 and outputting the same, thereby enabling more satisfactory health care which is closely tied with a user's daily life and enabling health care without a user noticing at that time, resulting in realizing improvement in facility in use.

In the health care system 1 according to the first exemplary embodiment, by setting up the health care system 1 by the biological information management device (portable communication terminal 20) which obtains user authentication from the biological information obtaining devices 10A and 10B by short-distance radio communication and receives a user's biological information measured and the health care information providing device (health care information providing server 30) which is connected to the biological information management device via the communication network 50 to receive biological information transmitted from the biological information management device, process the same to generate health care information and transmit the information to the biological information management device as a requesting source, more satisfactory health care which is closely tied with a user's daily life can be realized to enable health care without a user noticing and realize improvement in facility in use.

The reason is that the biological information management device (portable communication terminal 20) receives a user's biological information measured after obtaining user authentication through short-distance radio communication from the biological information obtaining devices 10A and 10B, automatically transmits the same to the health care information providing server 30 via the communication network 50, receives health care information processed by an analysis based on the biological information and transmitted from the health care information providing server 30 and automatically outputs the information.

In the health care system 1 according to the first exemplary embodiment, a user's heath care will be even more satisfactory by the cooperation with the present health care information providing service also at a fitness club, a restaurant, a shop or the like outside the house. Behavior tracing and safety check aiming at a user's health care are also enabled by managing a user's position information obtained by positioning arithmetic operation with correlated biological information.

The function held by the control unit 21 of the portable communication terminal 30 shown in Fig. 4 may be realized all in software or at least a part of the same may be realized in hardware. For example, data processing executed by the control unit 21 to receive a user's biological information measured after obtaining user authentication through short-distance radio communication from the biological information obtaining device 10A (10B), transmit the received biological information to the health care information providing server 30 via the communication network 50, receive health care information processed by an analysis based on the biological information and transmitted from the health care information providing server 30, and output the same may be realized on a computer by one or a plurality of programs, or at least a part of the processing may be realized in hardware.

Although the present invention has been described with respect to the preferred exemplary embodiment and mode of implementation in the foregoing, the present invention is not necessarily limited to the above-described exemplary embodiment and mode of implementation but can be modified without departing from the scope of its technical idea.

### INCORPORATION BY REFERENCE

This application is based upon and claims the benefit of priority from Japanese patent application No. 2009-123498, filed on May 21, 2009, the disclosure of which is incorporated herein in its entirety by reference.

## Claims

1. A biological information management device, comprising:
a control unit which is connected to a biological information obtaining device via short-distance radio communication and connected to a health care information providing device via a communication network;
wherein said control unit receives biological information of a user measured after obtaining user authentication by said short-distance radio communication from said biological information obtaining device, and transmits said biological information received to said health care information providing device via said communication network, receives health care information which is processed by an analysis based on said biological information and transmitted from said health care information providing device and outputs the received information.

2. The biological information management device according to claim 1, wherein said control unit obtains user authentication by short-distance radio communication from a health care information intermediating device further connected via said communication network, receives health care information transmitted from said health care information providing device via said health care information intermediating device and outputs the received information.

3. The biological information management device according to claim 1 or claim 2, wherein said control unit executes positioning arithmetic operation based on received current position information of a user, correlates the operation result with said obtained biological information and transmits the obtained result to said health care information providing device via said communication network.

4. The biological information management device according to any one of claim 1 through claim 3, wherein said control unit generates and outputs display information for displaying health care information transmitted from said health care information providing device or said health care information intermediating device on at least a part of a screen.

5. A health care system using a biological information management device, comprising:
a biological information obtaining device;
a biological information management device which receives biological information of a user measured after obtaining user authentication by short-distance radio communication from said biological information obtaining device; and
a health care information providing device connected to said biological information management device via a communication network to receive said biological information transmitted from said biological information management device, generate health care information by processing and transmit the generated information to said biological information management device as a requesting source.

6. The health care system using a biological information management device according to claim 5, further comprising:
a health care information intermediating device connected to said health care information providing device via said communication network to, when user authentication from said biological information management device by short-distance radio communication is established, intermediate health care information based on the biological information of said user transmitted from said health care information providing device and transmit the obtained information.

7. A method of reading health care information in a health care system including a biological information obtaining device, a health care information providing device and a biological information management device which is connected to said biological information obtaining device via short-distance radio communication and connected to said health care information providing device via a communication network, wherein
said biological information management device includes
a first step of receiving biological information of a user measured after obtaining user authentication by the short-distance radio communication from said biological information obtaining device, and
a second step of transmitting said biological information received to said health care information providing device via said communication network,
said health care information providing device includes
a third step of processing biological information received from said biological information management device by an analysis and transmitting the generated health care information to said biological information management device or said health care information intermediating device, and
said biological information management device includes
a fourth step of receiving health care information transmitted from said health care information providing device to cause a user to read the information, or obtaining user authentication by short-distance radio communication from said health care information intermediating device and receiving health care information transmitted from said health care information intermediating device to cause the user to read the information.

8. A biological information management program executed on a computer which realizes a biological information management device connected to a biological information obtaining device via short-distance radio communication and connected to a health care information providing device via a communication network, which causes the computer to execute:
a biological information reception processing of receiving biological information of a user measured after obtaining user authentication by said short-distance radio communication from said biological information obtaining device, and
a control processing of transmitting said biological information received to said health care information providing device via said communication network, receiving health care information which is processed by analyzing said biological information and transmitted from said health care information providing device and outputting the received information.

9. The biological information management program according to claim 8,
wherein in said control processing, user authentication is obtained by short-distance radio communication from a health care information intermediating device further connected via said communication network to receive health care information transmitted from said health care information providing device via said health care information intermediating device and output the received information.

10. The biological information management program according to claim 8 or claim 9, wherein in said control processing, positioning arithmetic operation is executed based on a user's current position information received to correlate the operation result with said obtained biological information and transmit the obtained result to said health care information providing device via said communication network.

11. The biological information management program according to any one of claim 8 through claim 10, wherein in said control processing, display information is generated and output for displaying health care information transmitted from said health care information providing device or said health care information intermediating device on at least a part of a screen.
